# EUROPEAN PATENT SPECIFICATION

(11) **EP 4 046 591 B1**
(45) Date of publication and mention of the grant of the patent: **10.04.2024**
(21) Application number: 22157848.7
(22) Date of filing: 21.02.2022
(51) Int. Cl.: A61C 9/00, A61C 5/70, A61C 13/00, G16H 30/40, A61C 5/77

(54) **METHOD FOR OBTAINING SUBGINGIVAL MARGINAL SHAPE FOR PROSTHESIS DESIGN NON-INVASIVELY**
VERFAHREN ZUR NICHT-INVASIVEN GEWINNUNG DER SUBGINGIVALEN RANDFORM FÜR DEN ENTWURF VON PROTHESEN
PROCÉDÉ PERMETTANT D'OBTENIR UNE FORME MARGINALE SOUS-GINGIVALE POUR UNE CONCEPTION DE PROTHÈSE DE MANIÈRE NON INVASIVE

(30) Priority: 19.02.2021 KR 20210022390
(43) Date of publication of application: 24.08.2022
(73) Proprietor: Huvitz Co., Ltd., Anyang-si, 14055 (KR); Ossvis Co., Ltd., Anyang-si, Gyeonggi-do 14055 (KR)
(72) Inventor: JEONG, Hyo Sang, Buk-gu, Daegu (KR); HAN, Su Min, Seoul (KR); LEE, Weon Joon, Anyang-si Gyeonggi-do 14055 (KR)
(74) Representative: Wellburn, Daniel

(56) References cited:
- US-A1- 2012 322 025
- LEE SANGBONG ET AL: "Fabrication of Dental Crown by Optical Coherence Tomography: A Pilot Study", IEEE ACCESS, IEEE, USA, vol. 8, 4 August 2020 (2020-08-04), pages 144969-144975, XP011804684, DOI: 10.1109/ACCESS.2020.3014315 [retrieved on 2020-08-14]

## Description

### Field

The present disclosure relates to a method for obtaining a subgingival marginal shape for a prosthesis design non-invasively, and more specifically, a method for obtaining a subgingival marginal shape for a prosthesis design non-invasively that is capable of obtaining a shape of a prepared tooth for the prosthesis design using a subgingival marginal shape obtained by means of an optical coherence tomography (OCT) scanner and a shape of a prepared tooth surface obtained by means of an intraoral scanner in a process of a prosthetic treatment.

### Background

A dental prosthesis is a dental appliance that replaces a missing tooth or covers up tooth defects which decrease oral function, such as a missing tooth or abnormality in shape, to thus restore the original function of the tooth.

For example, if a tooth is decayed, gum disease occurs, or a tooth is lost or broken due to an injury or accident, a prosthetic treatment is performed to restore the tooth having a problem with a normal, full or original tooth shape.

The dental prosthesis can be largely classified into a fixed prosthesis and a removable prosthesis. The fixed prosthesis is needed when a tooth is decayed or broken, without being lost, and thus requires reinforcement or when a tooth is lost to fix an artificial tooth to a position of the lost tooth. Contrarily, the removable prosthesis is detachably fixed, like a denture.

The fixed prosthesis includes a crown put on a tooth, a bridge connecting artificial teeth, and a resin, an inlay, or an onlay that is filled in a cut or damaged portion of a tooth.

In the process of prosthetic treatment, tooth preparation is needed for mounting the crown or bridge. The tooth preparation allows the prosthesis to be mountable and has an influence on a coupling force between the tooth and the prosthesis. The tooth preparation is an important process involving cutting the tooth so that the prosthesis cannot be lost or broken.

A conventional intraoral scanner obtains a shape of a prepared tooth only from a surface shape of the prepared tooth which is 'visible' to the intraoral scanner. Accordingly, some of the prepared tooth is hidden from the intraoral scanner by the gingiva and thus the intraoral scanner is unable to accurately obtain a subgingival marginal shape.
Lee Sangbong et. al. "Fabrication of Dental Crown by Optical Coherence Tomography: A Pilot Study" EEE ACCESS, IEEE, USA, vol. 8, 4 August 2020 (2020-08-04), pages 144969-144975 describes digital impressions which have been studied for better gingival retraction in including the under subgingival finish line condition.

### SUMMARY

Accordingly, the present disclosure has been made in view of the above-mentioned problems occurring in the related art, and it is an object of the present disclosure to provide a method for obtaining a subgingival marginal shape for a prepared tooth and a corresponding prosthesis design non-invasively.

It is another object of the present disclosure is to provide a method for obtaining a subgingival marginal shape for a prosthesis design non-invasively that is capable of obtaining a surface shape of a prepared tooth by means of an intraoral scanner and a subgingival marginal shape of the prepared tooth by means of an OCT scanner so that the prepared tooth surface shape is merged with the subgingival marginal shape to thus acquire the entire shape of the prepared tooth.

To accomplish the above-mentioned objects, there is provided a method for obtaining a subgingival marginal shape for a prosthesis design non-invasively by means of an optical coherence tomography (OCT) scanner for obtaining a subgingival marginal shape and an intraoral scanner for obtaining a tooth surface shape, the method including the steps of: obtaining a surface shape of a prepared tooth by means of the intraoral scanner; obtaining the subgingival marginal shape (crown margin line) of the prepared tooth by means of the OCT scanner; and merging the surface shape of the prepared tooth with the subgingival marginal shape to obtain a final three-dimensional shape of the prepared tooth.

According to embodiments, desirably, the OCT scanner may be an OCT system.

According to embodiments, desirably, the step of obtaining the subgingival marginal shape (crown margin line) of the prepared tooth may include the step of marking margin lines on the slice images of the OCT scanner that are repeatedly retrieved from the first slice position of a prepared tooth area to the last slice position thereof.

According to embodiments, desirably, the method may further include the step of connecting the marked margin line to the crown margin line.

According to embodiments, desirably, the method may further include the step of transforming the margin line and the crown margin line into STL/PLY formats.

### BRIEF DESCRIPTION OF THE DRAWINGS

The above and other objects, features and advantages will be apparent from the following detailed description of embodiments in conjunction with the accompanying drawings, in which:
FIG. 1 is a schematic showing a method for obtaining a subgingival marginal shape for a prosthesis design non-invasively according to embodiments; and
FIG 2A is a schematic showing a method for obtaining a final shape of a prepared tooth by merging a prepared tooth surface shape and a crown marginal shape according to embodiments.
FIG. 2B is a flowchart showing a process of obtaining a final shape of a prepared tooth by merging a prepared tooth surface shape and a crown marginal shape according to embodiments.

### DETAILED DESCRIPTION OF EMBODIMENTS

Objects, characteristics and advantages of the claimed invention will be more clearly understood from the detailed description as will be described below and the attached drawings. It is to be understood that the disclosed embodiments are merely exemplary and the claimed invention can be embodied in various forms. Embodiments are described in detail so that the skilled person can more easily put the invention into practice. However, the embodiments do not limit the claims. The invention is defined by the scope of the appended claims.

In the description, the thicknesses of the lines or the sizes of the components shown in the drawing may be magnified for the clarity and convenience of the description. In the description, further, the corresponding parts in the embodiments are indicated by corresponding reference numerals.

A term 'and/or' includes a combination of a plurality of relevant and described items or any one of a plurality of related and described items. An expression referencing a singular value additionally refers to a corresponding expression of the plural number, unless explicitly limited otherwise by the context. In this application, terms, such as "comprise", "include", or 'have", are intended to designate those characteristics, numbers, steps, operations, elements, or parts which are described in the specification, or any combination of them that exist.

Hereinafter, embodiments will be described and explained in detail with reference to the attached drawings.

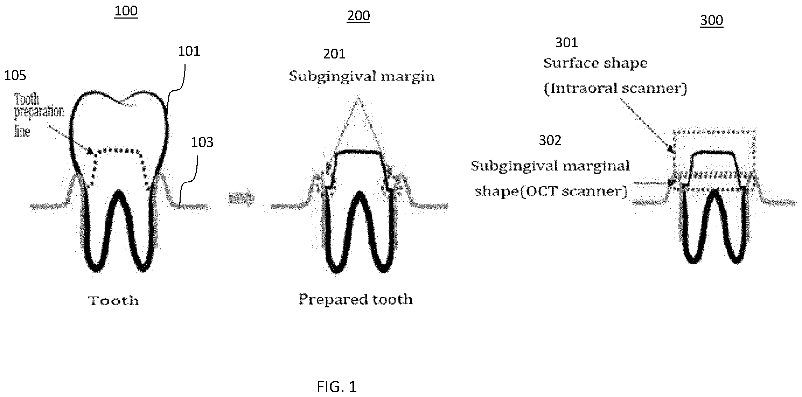

FIG. 1 is a schematic showing a method for obtaining a subgingival marginal shape for a prosthesis design non-invasively according to embodiments.

FIG. 1 shows a schematic cross-sectional view of a normal, full or original tooth 100 including lines indicating the cross-sectional shapes of the tooth outline 101, the gum outline 103 and the tooth preparation line 105. FIG. 1 also shows a schematic cross-sectional view of a prepared tooth 200 including indications of the location of a subgingival margin 201, and a schematic cross-sectional view of a prepared tooth 300 including indications of the location of the surface shape 301 and subgingival marginal shape of the tooth.

Referring to FIG. 1, a method for obtaining a subgingival marginal shape for a prosthesis design non-invasively according to embodiments is performed using both of an intraoral scanner for obtaining a tooth surface shape and an OCT scanner for obtaining a subgingival marginal shape.

In particular, the intraoral scanner serves to capture a tooth surface shape 301 of the prepared tooth. The surface shape 301 may be defined as the shape of surface of the prepared tooth which is (visible) above the gingiva. The intraoral scanner projects a light source of a projector onto a tooth surface and produces an image of the tooth surface using a camera to obtain the tooth surface shape 301. The detailed operation of an intraoral scanner is known to the skilled person and therefore a detailed description thereof is omitted.

The subgingival margin 201 is defined as the portion of the tooth under a boundary line defined by the gingiva (i.e. the gumline) at the time when the tooth is prepared. The surface of the tooth below the gumline is hidden from the intraoral scanner by the gingiva. Thus it is not usually possible to obtain the subgingival marginal shape 302 of the prepared tooth. However, the use of an OCT scanner allows the subgingival marginal shape 302 to be obtained through a tomography technology.

Accordingly, the OCT scanner can obtain the subgingival marginal shape 302. The subgingival marginal shape 302 may be defined as the shape of the prepared tooth which is in the subgingival margin 201. The subgingival marginal shape may also be referred to as the crown marginal shape.

Therefore, the tooth surface shape 301 obtained by the intraoral scanner is merged with the subgingival marginal shape 302 obtained by the OCT scanner to thus acquire a final shape of the prepared tooth for a prosthesis design upon a prosthesis treatment.

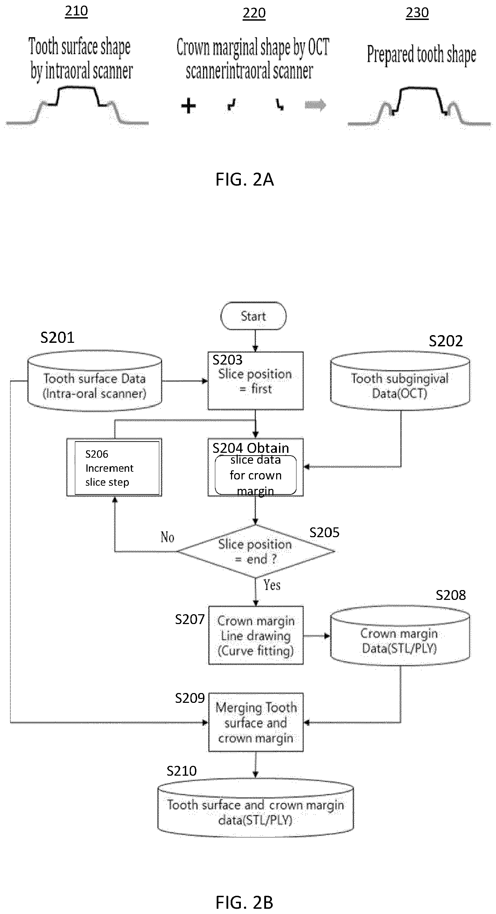

FIG 2A is a schematic showing a method for obtaining a final shape of a prepared tooth by merging a prepared tooth surface shape and a crown marginal shape according to embodiments.

FIG 2A shows a cross-sectional schematic of a tooth surface shape 210 obtained using an intraoral scanner, a cross-sectional schematic of a subgingival margin shape 220 (or crown marginal shape) obtained using an OCT scanner, and a cross-sectional schematic of a hybrid (or combined) shape 230 comprising a combination (e.g. an addition, or merging) of the tooth surface shape and the subgingival margin shape.

FIG. 2B is a flowchart showing a process of obtaining a final shape of a prepared tooth by merging a tooth surface shape and a crown marginal shape according to the present invention.

Referring to FIGs. 2A and 2B, at a first step S201 the tooth surface shape (tooth surface data) is obtained by an intraoral scanner. At a second and third step S202, S203, a slice image of the prepared tooth is obtained by an OCT scanner at a first slice position. The first slice position corresponds to the upper boundary of the subgingival margin area on the obtained tooth surface shape. Next, at a fourth to seventh step S204-S207, a slice image is obtained and a crown margin is marked in a slice image. In this case, the crown margin is a boundary surface of a prepared tooth such as the tooth preparation line 105 as shown in FIG. 1 below the gumline.

In a fifth and sixth step S205, S206, the slice images of the OCT scanner up to the last slice position of the tooth preparation area line are repeatedly retrieved. In a seventh step S207, a crown margin line is marked on the slice images of the OCT scanner. In this case, the crown margin line is in the subgingival margin. The crown margin line is marked by line drawing (curve fitting) the boundary of the prepared tooth in the subgingival margin based on the slice data. In an eighth step S208, crown margin data is obtained from the line drawing/curve fitting step and stored (e.g. in a memory).

Next, in a ninth step S209, the tooth surface shape obtained in the first step is connected to the crown margin line. That is, the boundary surface of the prepared tooth is connected to the crown margin line.

The margin portion (boundary surface of the prepared tooth) and the crown margin line (the subgingival margin) are transformed into data (for example, STL/PLY formats) available in a 3D tool S/W including a CAD.

Lastly, in a tenth step S210, the tooth surface shape data of the intraoral scanner and the crown margin data of the OCT scanner, as the transformed data, are merged with each other, thereby measuring the entire shape of the prepared tooth.

Therefore, the tooth surface shape obtained by the intraoral scanner is merged with the subgingival marginal shape obtained by the OCT scanner to thus acquire a final shape of the prepared tooth for a prosthesis design upon a prosthesis treatment.

As set forth in the foregoing, the present invention can obtain the subgingival marginal shape by means of the OCT scanner to acquire the entire shape of the prepared tooth for a good quality of prosthesis design.

The foregoing description of the embodiments of the invention has been presented for the purpose of illustration; it is not intended to be exhaustive or to limit the invention to the precise forms disclosed. Persons skilled in the relevant art can appreciate that many modifications and variations are possible in light of the above teachings. It is therefore intended that the scope of the invention be limited not by this detailed description, but rather by the claims appended hereto.
There is also provided the following alternative description of the Figures:
*FIG. 1* *is a concept view showing a method for obtaining a subgingival marginal shape for a prosthesis design non-invasively according to the present invention.*

*Referring to* *FIG. 1**, a method for obtaining a subgingival marginal shape for a prosthesis design non-invasively according to the present invention is performed using both of an intraoral scanner for obtaining a tooth surface shape and an OCT scanner for obtaining a subgingival marginal shape.*

*In specific, the intraoral scanner serves to capture a tooth surface shape. The intraoral scanner projects a light source of a projector onto a tooth surface and produces an image of the tooth surface by a camera to obtain the tooth surface shape.*

*The subgingival margin is a boundary line of a tooth under the gingiva (the gum) at the time when the tooth is prepared, and the intraoral scanner for obtaining the tooth surface shape is hidden by the gingiva to thus obtain no subgingival marginal shape. However, the OCT scanner obtains the subgingival marginal shape through a tomography technology.*

*Accordingly, the OCT scanner can obtain the subgingival marginal shape.*

*Therefore, the tooth surface shape obtained by the intraoral scanner is merged with the subgingival marginal shape obtained by the OCT scanner to thus acquire a final shape of the prepared tooth for a prosthesis design upon a prosthesis treatment.*

*FIG. 2A and 2B* *include a flowchart showing* a *process of obtaining a final shape* of a *prepared tooth by merging a tooth surface shape and a crown marginal shape according to the present invention.*

*Referring to* *FIGs. 1**,* *2A and 2B**, the tooth surface shape is obtained by the intraoral scanner, and a slice image of the OCT scanner at a first slice position corresponding to a tooth preparation area on the obtained tooth surface shape is retrieved. Next, a margin portion on the slice image is marked. In this case, the margin portion is a boundary surface of a prepared tooth line.*

*After that, the slice images of the OCT scanner up to the last slice position of the tooth preparation area are repeatedly retrieved, and a crown margin line on the slice images of the OCT scanner is marked. In this case, the crown margin line is the subgingival margin.*

*Next, the marked portion is connected to the crown margin line. That is, the boundary surface of the prepared tooth line is connected to the subgingival margin.*

*The margin portion (boundary surface of the prepared tooth line) and the crown margin line (the subgingival margin) are transformed into data (for example, STL*/*PLY formats) available in a 3D tool S*/*W including a CAD.*

*Lastly, the tooth surface shape data of the intraoral scanner and the crown margin data of the OCT scanner, as the transformed data, are merged with each other, thereby measuring the entire shape of the prepared tooth.*

*Therefore, the tooth surface shape obtained by the intraoral scanner is merged with the subgingival marginal shape obtained by the OCT scanner to thus acquire a final shape of the prepared tooth for a prosthesis design upon a prosthesis treatment.*

As *set forth in the foregoing, the present invention can obtain the subgingival marginal shape by means of the OCT scanner to acquire the entire shape of the prepared tooth for a good quality of prosthesis design.*

## Claims

1. A method for non-invasively obtaining a shape of a prepared tooth, the method comprising the steps of:
obtaining a tooth surface shape (210) of a prepared tooth (200) by means of an intraoral scanner;
obtaining a subgingival marginal shape (302) or crown margin line (103) of the prepared tooth by means of an optical coherence tomography "OCT" scanner; and
merging the surface shape of the prepared tooth with the subgingival marginal shape to obtain a three-dimensional shape of the prepared tooth.

2. The method according to claim 1, wherein the OCT scanner is an OCT system.

3. The method according to claim 1 or 2, wherein the step of obtaining the subgingival marginal shape or crown margin line of the prepared tooth comprises the step of marking at least one margin line onto slice images of the OCT scanner that are repeatedly retrieved from a first slice position of a prepared tooth area to a last slice position of the prepared tooth area.

4. The method according to claim 3, further comprising the step of connecting the marked margin line to the crown margin line.

5. The method according to claim 3 or 4, further comprising the step of transforming the margin line and the crown margin line into STL/PLY formats.

6. A method for non-invasively obtaining a shape for a prosthesis design, the method comprising the steps of: obtaining a three-dimensional shape of a prepared tooth according to the method of any preceding claim, and obtaining the shape of the prosthesis using the obtained three-dimensional shape of the prepared tooth.

## Patentansprüche

1. Verfahren zur nicht-invasiven Gewinnung einer Form eines präparierten Zahns, wobei das Verfahren die folgenden Schritte umfasst:
Gewinnen einer Zahnoberflächenform (210) eines präparierten Zahns (200) mittels eines Intraoralscanners;
Gewinnen einer subgingivalen Randform (302) oder einer Zahnkronenrandlinie (103) des präparierten Zahns mittels eines Scanners für optische Kohärenztomografie, "OCT"; und
Zusammenführen der Oberflächenform des präparierten Zahns mit der subgingivalen Randform, um eine dreidimensionale Form des präparierten Zahns zu gewinnen.

2. Verfahren nach Anspruch 1, wobei der OCT-Scanner ein OCT-System ist.

3. Verfahren nach Anspruch 1 oder 2, wobei der Schritt des Gewinnens der subgingivalen Randform oder der Kronenrandlinie des präparierten Zahns den Schritt des Markierens mindestens einer Randlinie auf Schichtbildern des OCT-Scanners umfasst, die von einer ersten Schichtposition eines Bereichs des präparierten Zahns bis zu einer letzten Schichtposition des Bereichs des präparierten Zahns wiederholt abgerufen werden.

4. Verfahren nach Anspruch 3, ferner umfassend den Schritt des Verbindens der markierten Randlinie mit der Kronenrandlinie.

5. Verfahren nach Anspruch 3 oder 4, ferner umfassend den Schritt des Umwandelns der Randlinie und der Kronenrandlinie in STL/PLY-Formate.

6. Verfahren zur nicht-invasiven Gewinnung einer Form für den Entwurf von Prothesen, wobei das Verfahren die folgenden Schritte umfasst: Gewinnen einer dreidimensionalen Form eines präparierten Zahns gemäß dem Verfahren nach einem der vorhergehenden Ansprüche und Gewinnen der Form der Prothese unter Verwendung der gewonnenen dreidimensionalen Form des präparierten Zahns.

## Revendications

1. Procédé permettant d'obtenir de manière non invasive une forme d'une dent préparée, le procédé comprenant les étapes de :
l'obtention d'une forme de surface de la dent (210) d'une dent préparée (200) au moyen d'un scanner intra-oral ;
l'obtention d'une forme marginale sous-gingivale (302) ou d'une ligne de marge de couronne (103) de la dent préparée au moyen d'un scanner de tomographie par cohérence optique "OCT" ; et
la fusion de la forme de surface de la dent préparée avec la forme marginale sous-gingivale pour obtenir une forme tridimensionnelle de la dent préparée.

2. Procédé selon la revendication 1, le scanner OCT étant un système OCT.

3. Procédé selon la revendication 1 ou 2, l'étape d'obtention de la forme marginale sous-gingivale ou de la ligne de marge de couronne de la dent préparée comprenant l'étape de marquage d'au moins une ligne de marge sur des images de tranches du scanner OCT qui sont récupérées de manière répétée depuis une première position de tranche d'une zone de dent préparée jusqu'à une dernière position de tranche de la zone de dent préparée.

4. Procédé selon la revendication 3, comprenant en outre l'étape de connexion de la ligne de marge marquée à la ligne de marge de couronne.

5. Procédé selon la revendication 3 ou 4, comprenant en outre l'étape de transformation de la ligne de marge et la ligne de marge de couronne en formats STL/PLY.

6. Procédé d'obtention non invasive d'une forme pour la conception d'une prothèse, le procédé comprenant les étapes suivantes : obtention d'une forme tridimensionnelle d'une dent préparée conformément au procédé selon n'importe quelle revendication précédente, et obtention de la forme de la prothèse à l'aide de la forme tridimensionnelle obtenue de la dent préparée.
